# EUROPEAN PATENT APPLICATION

(11) **EP 3 756 540 A1**
(43) Date of publication of application: **30.12.2020**
(21) Application number: 19757339.7
(22) Date of filing: 04.02.2019
(51) Int. Cl.: A61B 5/02, A61B 5/00

(54) **ELECTRONIC DEVICE, ESTIMATION SYSTEM, ESTIMATION METHOD, AND ESTIMATION PROGRAM**

(30) Priority: 22.02.2018 JP 2018030116
(71) Applicant: Kyocera Corporation, Fushimi-ku, Kyoto-shi, Kyoto 612-8501 (JP)
(72) Inventor: AJIMA Hiromi, Kyoto-shi, Kyoto 612-8501 (JP)
(74) Representative: Viering, Jentschura & Partner mbB Patent- und Rechtsanwälte
(86) International application number: PCT/JP2019/003867
(87) International publication number: WO 2019/163501

(57) **Abstract**

Provided is an electronic device 100 including a sensor unit 130 configured to acquire a pulse wave of a subject and a controller 143 configured to estimate, using an estimation formula created based on a preprandial pulse wave and a postprandial pulse wave and blood glucose level, a postprandial blood glucose level of the subject based on a difference between the preprandial pulse wave and the postprandial pulse wave of the subject acquired by the sensor unit 130.

## Description

### CROSS-REFERENCE TO RELATED APPLICATION

This application claims priority to and benefit of Japanese Patent Application No. 2018-030116 filed on February 22, 2018, the entire contents of which are incorporated herein by reference.

### TECHNICAL FIELD

This disclosure relates to an electronic device, an estimation system, an estimation method and an estimation program that estimate the health condition of a subject from measured biological information.

### BACKGROUND

In the related art, measurement of blood component and measurement of blood fluidity have been made as a means of estimating the health condition of a subject (user). These measurements are made by using the blood collected from the subject. Further, an electronic device that measures the biological information from a measured part such as a wrist of the subject is known. For example, the Patent Literature 1 (PTL 1) discloses an electronic device that measures the pulse of the subject when worn on a wrist of the subject.

### CITATION LIST

### Patent Literature

PLT 1: JP2002-360530(A)

### SUMMARY

One aspect of an electronic device includes a sensor unit configured to acquire a pulse wave of a subject and a controller configured to estimate, using an estimation formula created based on a preprandial pulse wave and a postprandial pulse wave and blood glucose level, a postprandial blood glucose level of the subject based on a difference between a preprandial pulse wave and a postprandial pulse wave of the subject acquired by the sensor unit.

Another aspect of the electronic device includes a sensor unit configured to acquire a pulse wave of a subject and a controller configured to estimate, using an estimation formula created based on a preprandial pulse wave and a postprandial pulse wave and lipid level, a postprandial lipid level of the subject based on a difference between a preprandial pulse wave and a postprandial pulse wave of the subject acquired by the sensor unit.

One aspect of an estimation system is an estimation system including an electronic device and an information processor communicatively connected to each other. The electronic device includes a sensor unit configured to acquire a pulse wave of a subject. The information processor includes a controller configured to estimate, using an estimation formula created based on a preprandial pulse wave and a postprandial pulse wave and blood glucose level, a postprandial blood glucose level of the subject based on a difference between a preprandial pulse wave and a postprandial pulse wave of the subject acquired by the sensor unit.

Another aspect of the estimation system is an estimation system including an electronic device and an information processor communicatively connected to each other. The electronic device includes a sensor unit configured to acquire a pulse wave of the subject. The information processor includes a controller configured to estimate, using an estimation formula created based on a preprandial pulse wave and a postprandial pulse wave and lipid level, a postprandial lipid level of the subject based on a difference between a preprandial pulse wave and a postprandial pulse wave of the subject acquired by the sensor unit.

One aspect of an estimation method is an estimation method executed by an electronic device. The estimation method includes the steps of: acquiring a pulse wave of a subject; and estimating, using an estimation formula created based on a preprandial pulse wave and a postprandial pulse wave and blood glucose level, a postprandial blood glucose level of the subject based on a difference between a preprandial pulse wave and a postprandial pulse wave of the subject acquired.

Another aspect of the estimation method is an estimation method executed by an electronic device. The estimation method includes the steps of: acquiring a pulse wave of a subject; and estimating, using an estimation formula created based on a preprandial pulse wave and a postprandial pulse wave and lipid level, a postprandial lipid level of the subject based on a difference between a preprandial pulse wave and a postprandial pulse wave of the subject acquired.

One aspect of an estimation program causes an electronic device to execute the steps of: acquiring a pulse wave of a subject; and estimating, using an estimation formula created based on a preprandial pulse wave and a postprandial pulse wave and blood glucose level, a postprandial blood glucose level of the subject based on a difference between a preprandial pulse wave and a postprandial pulse wave of the subject acquired.

Another aspect of the estimation program causes an electronic device to execute the steps of: acquiring a pulse wave of a subject; and estimating, using an estimation formula created based on a preprandial pulse wave and a postprandial pulse wave and lipid level, a postprandial lipid level of the subject based on a difference between a preprandial pulse wave and a postprandial pulse wave of the subject acquired.

One aspect of an electronic device includes a sensor unit configured to acquire a pulse wave of a subject and a controller configured to estimate, using an estimation formula created based on a preprandial pulse wave and a postprandial pulse wave and blood glucose level of the subject, a blood glucose level of the subject based on a difference between a preprandial pulse wave and a pulse wave at any timing of the subject acquired by the sensor unit.

Another aspect of the electronic device includes a sensor unit configured to acquire a pulse wave of a subject and a controller configured to estimate, using an estimation formula created based on a preprandial pulse wave and a postprandial pulse wave and lipid level of the subject, a lipid level of the subject based on a difference between a preprandial pulse wave and a pulse wave at any timing of the subject acquired by the sensor unit.

### BRIEF DESCRIPTION OF THE DRAWINGS

In the accompanying drawings:
FIG. 1 is a schematic diagram illustrating a schematic configuration of an example of an electronic device according to an embodiment;
FIG. 2 is a cross-sectional view illustrating a schematic configuration of the electronic device in FIG. 1;
FIG. 3 is a diagram illustrating an example of the electronic device in FIG. 1 during use;
FIG. 4 is a schematic external perspective view of an example of the electronic device according to an embodiment;
FIG. 5 is a schematic view illustrating a state where the electronic device in FIG. 4 is worn;
FIG. 6 is a schematic view illustrating an exterior portion and a sensor unit of the electronic device in a front view in FIG. 4;
FIG. 7 is a schematic view schematically illustrating a positional relationship between a wrist of a subject and a first arm of a sensor unit in a front view;
FIG. 8A is a schematic view schematically illustrating a positional relationship among a wrist of the subject, the first arm of the sensor unit and the exterior portion of a measurement unit in a front view;
FIG. 8B is a schematic view schematically illustrating a positional relationship among a wrist of the subject, the first arm of the sensor unit and the exterior portion of the measurement unit in a front view;
FIG. 9 is a function block diagram of the electronic device;
FIG. 10 is a diagram illustrating an example of an estimation method based on a change in a pulse wave in the electronic device;
FIG. 11 is a diagram illustrating an example of an acceleration pulse wave;
FIG. 12 is a diagram illustrating an example of a pulse wave acquired by the sensor unit;
FIG. 13A is a diagram illustrating another example of the estimation method based on a change in a pulse wave in the electronic device;
FIG. 13B is a diagram illustrating still another example of the estimation method based on a change in a pulse wave in the electronic device;
FIG. 14 is a flowchart for creating an estimation formula used by the electronic device in FIG. 1;
FIG. 15 is a diagram illustrating an example of a neural network regression analysis;
FIG. 16 is a flowchart for estimating, using an estimation formula, a postprandial blood glucose level of the subject;
FIG. 17 is a diagram illustrating a comparison between the estimated postprandial blood glucose level and the measured postprandial blood glucose level;
FIG. 18 is a diagram illustrating a comparison between the estimated postprandial blood glucose level and the measured postprandial blood glucose level;
FIG. 19 is a flowchart for estimating a postprandial blood glucose level of the subject by using a plurality of estimation formulas;
FIG. 20 is a flowchart for creating an estimation formula used by an electronic device according to a second embodiment;
FIG. 21 is a flowchart for estimating, using an estimation formula created by the flow in FIG. 20, a postprandial lipid level of the subject;
FIG. 22 is a schematic diagram illustrating a schematic configuration of a system according to an embodiment; and
FIG. 23 is a diagram illustrating an example of pulse wave.

### DETAILED DESCRIPTION

The method of blood sampling is painful and therefore difficult to be used on a daily basis to estimate the health condition of the subject. Further, in the method of wearing an electronic device configured to measure the biological information on a wrist, an object to be measured is conventionally limited to the pulse, and it is impossible to estimate the health condition of the subject except for the pulse. It is preferable that the health condition of the subject can be easily estimated.

Embodiments will be described in detail below with reference to the drawings.

### (First embodiment)

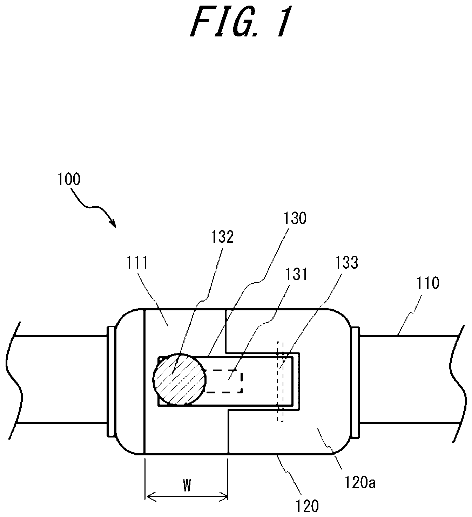

FIG. 1 is a schematic diagram illustrating a schematic configuration of Example 1 of an electronic device according to an embodiment. An electronic device 100 according to Example 1 illustrated in FIG. 1 includes an attaching portion 110 and a measurement unit 120. FIG. 1 is a diagram observing the electronic device 100 according to Example 1 from the back surface 120a that comes in contact with a measured part.

The electronic device 100 measures the biological information of the subject while the subject wears the electronic device 100. The biological information measured by the electronic device 100 includes a pulse wave of the subject. In an embodiment, the electronic device 100 of Example 1 may acquire a pulse wave while being worn on a wrist of the subject.

In an embodiment, the attaching portion 110 is a straight and elongated band. Pulse wave measurement is performed, for example, in a state in which the subject wraps the attaching portion 110 of the electronic device 100 around his/her wrist. More specifically, the subject wraps the attaching portion 110 around his/her wrist so that the back surface 120a of the measurement unit 120 is in contact with the measured part and then measures the pulse wave. The electronic device 100 measures the pulse wave of blood flowing through the ulnar artery or the radial artery of the subject.

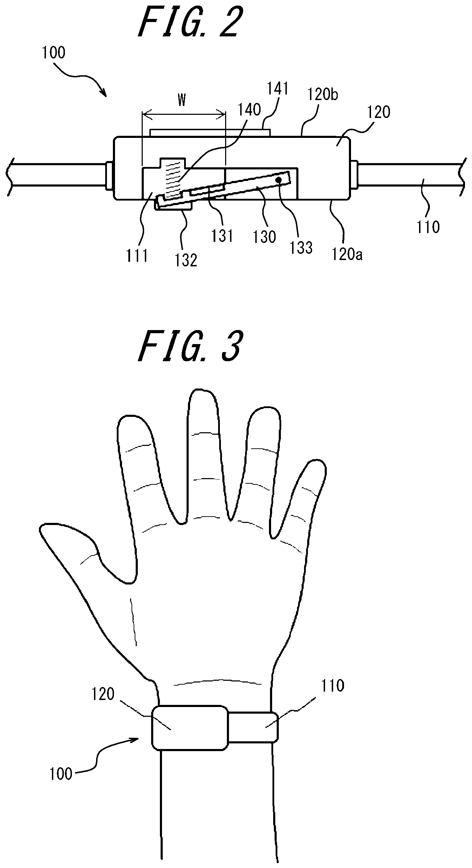

FIG. 2 is a cross-sectional diagram of the electronic device 100 according to Example 1. FIG. 2 illustrates the measurement unit 120 and the attaching portion 110 around the measurement unit 120.

The measurement unit 120 has the back surface 120a that comes in contact with the wrist of the subject when worn and a surface 120b on an opposite side from the back surface 120a. The measurement unit 120 has an opening 111 in the back surface 120a side. The sensor unit 130 has a first end that comes in contact with the wrist of the subject and a second end that comes in contact with the measurement unit 120 when the electronic device 100 of Example 1 is worn. In a state in which an elastic body 140 is not compressed, the first end of the sensor unit 130 protrudes from the opening 111 to the back surface 120a side. The first end of the sensor unit 130 has a pulse pad 132. The first end of the sensor unit 130 is displaceable in a direction nearly substantially perpendicular to the plane of the back surface 120a. The second end of the sensor unit 130 is in contact with the measurement unit 120 through a shaft 133.

The first end of the sensor unit 130 is in contact with the measurement unit 120 through the elastic body 140. The first end of the sensor unit 130 is displaceable relative to the measurement unit 120. The elastic body 140 includes, for example, a spring. The elastic body 140 is not limited to a spring, and may be any other elastic body such as a resin or a sponge. Further, instead of the elastic body 140 or together with the elastic body 140, a biasing mechanism such as a torsion coil spring may be provided to a rotary shaft 133 of the sensor unit 130 so that the pulse pad 132 of the sensor unit 130 is brought into contact with the measured part, which is an object to be measured of the pulse wave of the blood of the subject.

It is to be noted that a controller, a memory, a communication interface, a power source, a notification interface and a circuit that operates them, a cable for connection may be disposed at the measurement unit 120.

The sensor unit 130 includes an angular velocity sensor 131 configured to detect the displacement of the sensor unit 130. The angular velocity sensor 131 detects the angular displacement of the sensor unit 130. Each sensor provided in the sensor unit 130 is not limited to the angular velocity sensor 131 and may, for example, be an acceleration sensor, an angle sensor, or some other types of motion sensor, or a plurality of these sensors.

The electronic device 100 of Example 1 includes an input interface 141 on the front surface 120b side of the measurement unit 120. The input interface 141 receives operation input by the subject, and includes, for example, operation buttons (operation keys). The input interface 141 may be configured, for example, as a touch screen.

FIG. 3 is a diagram illustrating an example of the electronic device 100 of Example 1 used by a subject. The subject wraps the electronic device 100 of Example 1 around his/her wrist and uses it. The electronic device 100 of Example 1 is worn in a state where the back surface 120a of the measurement unit 120 is in contact with the wrist. The position of the measurement unit 120 can be adjusted so that the pulse pad 132 is in contact with the position of the ulnar artery or the radial artery while the electronic device 100 of Example 1 is wrapped around the wrist.

In FIG. 3, while the electronic device 100 of Example 1 is worn, the first end of the sensor unit 130 is in contact with the skin over the radial artery, which is the artery on the thumb side of the left hand of the subject. The first end of the sensor unit 130 is in contact with the skin over the radial artery of the subject as a result of the elastic force applied by the elastic body 140 arranged between the measurement unit 120 and the sensor unit 130. The sensor unit 130 is displaced in accordance with the movement of the radial artery, that is, pulsation of the subject. The angular velocity sensor 131 detects displacement of the sensor unit 130 and acquires the pulse wave. The pulse wave refers to a waveform representation of the temporal change in volume of a blood vessel caused by inflow of blood, acquired from the body surface.

Referring again to FIG. 2, in a state in which the elastic body 140 is not compressed, the first end of the sensor unit 130 protrudes from the opening 111. When the subject wears the electronic device 100 of Example 1, the first end of the sensor unit 130 is in contact with the skin over the radial artery of the subject, and according to the pulsation, the elastic body 140 expands and contracts, and the first end of the sensor unit 130 is displaced. The elastic body 140 with an appropriate elastic modulus is used so that it can expand and contract according to the pulsation without inhibiting the pulsation. The opening width W of the opening 111 is greater than the vessel diameter, i.e., the radial artery diameter in an embodiment. By providing the opening 111 in the measurement unit 120, the back surface 120a of the measurement unit 120 does not compress the radial artery when the electronic device 100 of Example 1 is worn. Therefore, the electronic device 100 of Example 1 can acquire a pulse wave with little noise, and thus the measurement accuracy is improved.

FIG. 3 illustrates an example in which the electronic device 100 of Example 1 is worn on the wrist and a pulse wave of the radial artery is acquired. However, for example, the electronic device 100 of Example 1 may acquire the pulse wave of blood flowing through a carotid artery in the neck of the subject. More specifically, the subject may press the pulse pad 132 lightly against the position of the carotid artery to measure the pulse wave. The subject may also wrap the electronic device 100 of Example 1 around his/her neck so that the pulse pad 132 is at the position of the carotid artery.

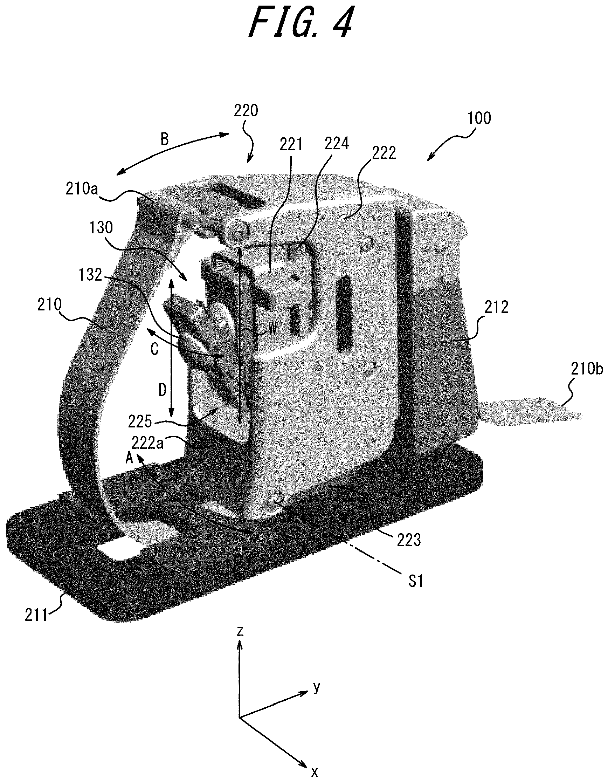

FIG. 4 is a schematic external perspective view of Example 2 of the electronic device according to an embodiment. The electronic device 100 of Example 2 illustrated in FIG. 4 includes an attaching portion 210, a base portion 211, a fixing portion 212 attached to the base portion 211 and a measurement unit 220.

In this embodiment, the base portion 211 is formed in a substantially rectangular flat plate shape. In this specification, as illustrated in FIG. 4, explanation will be given below by defining the short side direction of the flat plate shaped base portion 211 as the x-axis direction, the long side direction of the flat plate shaped base portion 211 as the y-axis direction and the orthogonal direction of the flat plate shaped base portion 211 as the z-axis direction. Although a part of the electronic device 100 of Example 2 is configured movable as described herein, when the direction of the electronic device 100 of Example 2 is described, unless otherwise mentioned, x, y and z-axis directions in the state illustrated in FIG. 4 will be indicated. Further, herein the z-axis positive direction is the upward direction, the z-axis negative direction is the downward direction and the x-axis positive direction is the front of the electronic device 100 of Example 2.

The electronic device 100 of Example 2 measures the biological information of the subject in a state where the subject wears the electronic device 100 of Example 2 using the attaching portion 210. The biological information measured by the electronic device 100 of Example 2 is the pulse wave of the subject that can be measured by the measurement unit 220. Explanation will be given below assuming that the electronic device 100 of Example 2 is worn on the wrist of the subject to acquire the pulse wave, as an example.

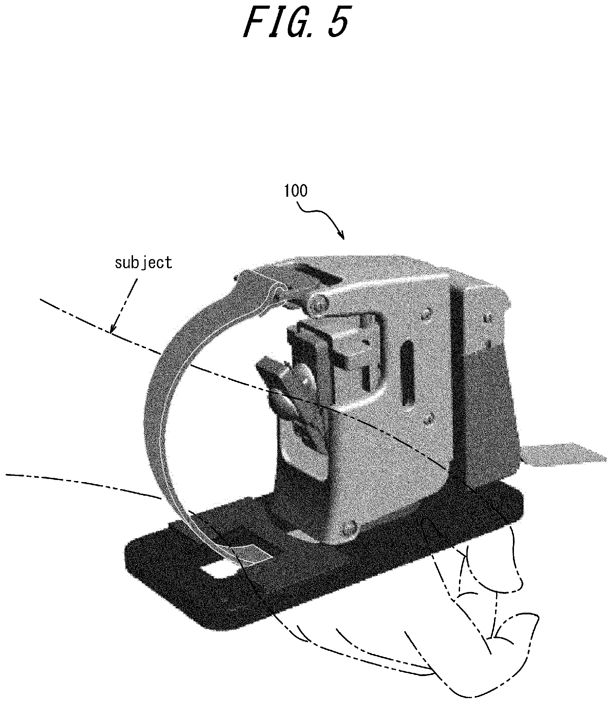

FIG. 5 is a schematic view illustrating a state where the subject wears the electronic device 100 of Example 2 in FIG. 4. The subject can wear the electronic device 100 as illustrated in FIG. 5 by passing his/her wrist through a space formed by the attaching portion 210, the base portion 211 and the measurement unit 220 and by fixing the wrist by the attaching portion 210. In the examples illustrated in FIGS. 4 and 5, the subject wears the electronic device 100 of Example 2 by passing his/her wrist through the space formed by the attaching portion 210, the base portion 211 and the measurement unit 220 along the x-axis direction toward the x-axis positive direction. The subject wears the electronic device 100 of Example 2 so that the pulse pad 132 of the measurement unit 220 described later is brought in contact with a position where the ulnar artery or the radial artery exists. The electronic device 100 of Example 2 measures the pulse wave of the blood flowing through the ulnar artery or the radial artery at the wrist of the subject.

The measurement unit 220 includes a body portion 221, an exterior portion 222 and a sensor unit 130. The sensor unit 130 is attached to the body portion 221. The measurement unit 220 is attached to the base portion 211 through a coupling portion 223.

The coupling portion 223 may be attached to the base portion 211 in a rotatable manner along the surface of the base portion 211. That is, in the example illustrated in FIG. 4, the coupling portion 223 may be attached rotatably to the base portion 211, as indicated by the arrow A, relative to the base portion 211 on the xy plane. In this case, the whole measurement unit 220 attached to the base portion 211 through the coupling portion 223 is rotatable relative to the base portion 211 on the xy plane.

The exterior portion 222 is coupled to the coupling portion 223 on the shaft S1 passing through the coupling portion 223. The shaft S1 is a shaft extending in the x-axis direction. Thus the exterior portion 222 is coupled to the coupling portion 223, and the exterior portion 222 is displaceable relative to the coupling portion 223 along a plane that intersects the xy plane on which the base portion 211 extends. That is, the exterior portion 222 can be inclined by a predetermined angle about the shaft S 1 on the xy plane on which the base portion 211 extends. For example, the exterior portion 222 can be displaced in a state where it rides on a surface such as the yz plane that is inclined at a predetermined angle relative to the xy plane. In this embodiment, the exterior portion 222 can be coupled to the coupling portion 223 rotatably about the shaft S1 on the yz plane orthogonal to the xy plane, as indicated by the arrow B in FIG. 4.

The exterior portion 222 has a contact surface 222a that comes in contact with the wrist of the subject when the electronic device 100 of Example 2 is worn. The exterior portion 222 may have an opening 225 on the contact surface 222a side. The exterior portion 222 may be configured such that it covers the body portion 221.

The exterior portion 222 may include, in the inside space, a shaft 224 extending in the z-axis direction. The body portion 221 has a hole through which the shaft 224 passes, and the body portion 221 is attached to the space inside the exterior portion 222 with the shaft 224 passed through the hole. That is, as indicated by the arrow C in FIG. 4, the body portion 221 is attached to the exterior portion 222 rotatably about the shaft 224 on the xy plane relative to the exterior portion 222. That is, the body portion 221 is attached to the exterior portion 222 rotatably along the xy plane, which is a surface of the base portion 211, relative to the exterior portion 222. Further, as indicated by the arrow D in FIG. 4, the body portion 221 is attached to the exterior portion 222 vertically displaceable relative to the exterior portion 222 along the shaft 224, that is the z-axis direction.

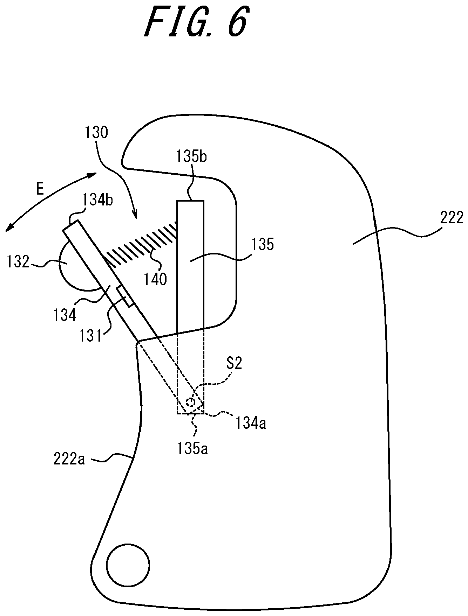

The sensor unit 130 is attached to the body portion 221. Here, the sensor unit 130 will be described in detail below with reference to FIG. 6. FIG. 6 is a schematic view illustrating the exterior portion 222 and the sensor unit 130 in a front view of the electronic device 100 of Example 2. In FIG. 6, a portion of the sensor unit 130 overlapping the exterior portion 222 in the front view is expressed by a broken line.

The sensor unit 130 includes a first arm 134 and a second arm 135. The second arm 135 is fixed to the body portion 221. A lower one end 135a of the second arm 135 is connected to one end 134a of the first arm 134. The first arm 134 is connected to the second arm 135 in such a manner that the other end 134b side is rotatable about one end 134a on the yz plane, as indicated by the arrow E in FIG. 6.

The other end 134b side of the first arm 134 is connected to the upper other end 135b side of the second arm 135 through the elastic body 140. In a state where the elastic body 140 is not compressed, the first arm 134 is supported by the second arm 135 with the other end 134b of the sensor unit 130 protruded from the opening 225 of the exterior portion 222 to the contact surface 222a side. The elastic body 140 is, for example, a spring. However, the elastic body 140 is not limited to a spring, and may be any other elastic body such as a resin or a sponge. Further, instead of the elastic body 140 or together with the elastic body 140, a biasing mechanism such as a torsion coil spring may be provided to a rotary shaft S2 of the first arm 134 so that the pulse pad 132 of the first arm 134 is brought into contact with the measured part, which is an object to be measured of the pulse wave of the blood of the subject.

The pulse pad 132 is coupled to the other end 134b of the first arm 134. When the electronic device 100 of Example 2 is worn, the pulse pad 132 is a portion that is brought into contact with a measured part, that is, an object to be measured of the pulse wave of the blood of the subject. In this embodiment, the pulse pad 132 is in contact with a position where the ulnar artery or the radial artery exists, for example. The pulse pad 132 may be made of a material that does not easily absorb changes in the body surface due to the pulse of the subject. The pulse pad 132 may be made of a material that does not easily cause the subject to feel pain when being in contact with the subject. For example, the pulse pad 132 may be formed of a cloth bag or the like in which beads are packed. The pulse pad 132 may be configured to be attachable to/detachable from the first arm 134, for example. For example, the subject may attach one pulse pad 132 of a plurality of pulse pads 132 having a variety of sizes and/or shapes to the first arm 134 according to the size and/or the shape of his/her wrist. In this manner, the subject can use the pulse pad 132 that fits the size and/or the shape of his/her wrist.

The sensor unit 130 includes an angular velocity sensor 131 configured to detect displacement of the first arm 134. It is sufficient for the angular velocity sensor 131 to detect the angular displacement of the first arm 134. The sensor unit 130 is not limited to the angular velocity sensor 131, and may be an acceleration sensor, an angular sensor, other motion sensors, or a plurality of these sensors, for example.

As illustrated in FIG. 5, in this embodiment, the pulse pad 132 is in contact with the skin over the radial artery, which is the artery on the thumb side of the right hand of the subject in a state where the electronic device 100 of Example 2 is worn. The pulse pad 132 disposed on the other end 134b side of the first arm 134 is in contact with the skin over the radial artery of the subject as a result of the elastic force applied by the elastic body 140 disposed between the second arm 135 and the first arm 134. The first arm 134 is displaced according to the movement of the radial artery, that is, the pulsation of the subject. The angular velocity sensor 131 acquires the pulse wave by detecting the displacement of the first arm 134. The pulse wave refers to a waveform representation of the temporal change in volume of a blood vessel caused by inflow of blood, acquired from the body surface.

As illustrated in FIG. 6, in a state in which the elastic body 140 is not compressed, the other end 134b of the first arm 134 protrudes from the opening 225. When the electronic device 100 is worn on the subject, the pulse pad 132 coupled to the first arm 134 is in contact with the skin over the radial artery of the subject, and according to the pulsation, the elastic body 140 expands and contracts, and the pulse pad 132 is displaced. The elastic body 140 with an appropriate elastic modulus is used so that it can expand and contract according to the pulsation without inhibiting the pulsation. The opening width W of the opening 225 is sufficiently greater than the vessel diameter, i.e., the radial artery diameter in this embodiment. By providing the opening 225 in the exterior portion 222, the contact surface 222a of the exterior portion 222 does not compress the radial artery when the electronic device 100 of Example 2 is worn. Therefore, the electronic device 100 of Example 2 can acquire a pulse wave with little noise, and thus the measurement accuracy is improved.

The fixing portion 212 is fixed to the base portion 211. The fixing portion 212 may include a fixing mechanism that fixes the attaching portion 210. The attaching portion 210 may include therein various functions used by the electronic device 100 of Example 2 to measure the pulse wave. For example, the fixing portion 212 may include an input interface, a controller, a power source, a memory, a communication interface, a notification interface and a circuit that operates them, a cable for connection and the like.

The attaching portion 210 is a mechanism used by the subject to secure his/her wrist to the electronic device 100 of Example 2. In the example illustrated in FIG. 4, the attaching portion 210 is an elongated band. In the example illustrated in FIG. 4, the attaching portion 210 is disposed such that one end 210a is coupled to the upper end of the measurement unit 220 and the other end 210b passes through the base portion 211 and is positioned on the y-axis positive direction side. For example, the subject passes his/her right wrist through the space formed by the attaching portion 210, the base portion 211 and the measurement unit 220, and pulls the other end 210b of the attaching portion 210 in the y-axis positive direction with his/her left hand while adjusting the pulse pad 132 to come in contact with the skin over the radial artery of the right wrist. The subject pulls the other end 210b such an extent that the right wrist is fixed to the electronic device 100 of Example 2, and in this state fixes the attaching portion 210 by a fixing mechanism of the fixing portion 212. In this manner, the subject can wear the electronic device 100 of Example 2 with his/her one hand (in this embodiment, the left hand). Further, the wearing state of the electronic device 100 of Example 2 can be stabilized by fixing his/her wrist to the electronic device 100 of Example 2 by using the attaching portion 210, which makes it difficult for the positional relationship between the wrist and the electronic device 100 of Example 2 to be changed during measurement. Thus stable measurement of the pulse wave is achieved, and the accuracy of the measurement is improved.

Next, movement of the movable portion of the electronic device 100 of Example 2 when the electronic device 100 of Example 2 is worn will be described.

When wearing the electronic device 100 of Example 2, the subject passes his/her wrist through the space formed by the attaching portion 210, the base portion 211 and the measurement unit 220 along the x-axis, as described above. At this time, since the measurement unit 220 is configured rotatably in the direction of the arrow A in FIG. 4 with respect to the base portion 211, the subject can pass through his/her wrist by rotating the measurement unit 220 in the direction indicated by the arrow A in FIG. 4. Since the measurement unit 220 is configured rotatably as described above, the subject can pass through his/her wrist while appropriately changing the direction of the measurement unit 220 according to the positional relationship between the subject and the electronic device 100 of Example 2. In this manner, according to the electronic device 100 of Example 2, the subject can easily wear the electronic device 100 of Example 2.

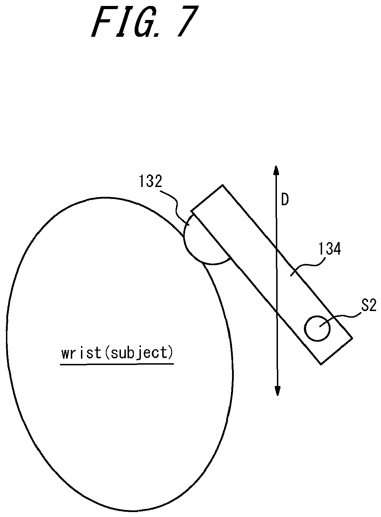

The subject passes his/her wrist through the space formed by the attaching portion 210, the base portion 211 and the measurement unit 220, and brings the pulse pad 132 into contact with the skin over the radial artery of his/her wrist. Here, since the body portion 221 is configured displaceably in the direction of the arrow D in FIG. 4, the first arm 134 of the sensor unit 130 coupled to the body portion 221 is also displaceable in the direction of the arrow D, which is in the z-axis direction, as illustrated in FIG. 7. Thus, the subject can displace the first arm 134 in the direction of the arrow D according to the size and the thickness of his/her wrist so that the pulse pad 132 comes in contact with the skin over the radial artery. The subject can fix the body portion 221 at the displaced position. In this manner, according to the electronic device 100 of Example 2, the position of the sensor unit 130 is easier to be adjusted to a position suitable for measurement. Thus, according to the electronic device 100 of Example 2, the measurement accuracy is improved. It is to be noted that, in the example illustrated in FIG. 4, although the body portion 221 has been described as being displaceable along the z-axis direction, the body portion 221 may not necessarily be configured to be displaceable along the z-axis direction. The body portion 221 may be configured so that its position can be adjusted depending on the size and the thickness of the wrist, for example. For example, the body portion 221 may be configured to be displaceable along a direction intersecting the xy plane, which is the surface of the base portion 211.

Here, when the pulse pad 132 is in contact with the skin over the radial artery in the direction orthogonal to the skin surface, the pulsation transmitted to the first arm 134 is increased. That is, when the displacement direction of the pulse pad 132 (the direction indicated by the arrow E in FIG. 3) is a direction orthogonal to the skin surface, the pulsation transmitted to the first arm 134 is increased, and acquisition accuracy of pulsation can be improved. In the electronic device 100 of Example 2, the body portion 221 and the sensor unit 130 coupled to the body portion 221 are configured rotatable about the shaft 224 with respect to the exterior portion 222, as indicated by the arrow C in FIG. 4. Thus, the subject can adjust the direction of the sensor unit 130 so that the displacement direction of the pulse pad 132 is orthogonal to the skin surface. That is, the electronic device 100 of Example 2 can adjust the direction of the sensor unit 130 so that the displacement direction of the pulse pad 132 is orthogonal to the skin surface. In this manner, according to the electronic device 100 of Example 2, the direction of the sensor unit 130 can be adjusted according to the shape of the wrist of the subject, and the change in the pulsation of the subject is transmitted more easily to the first arm 134. Thus, according to the electronic device 100 of Example 2, measurement accuracy is improved.

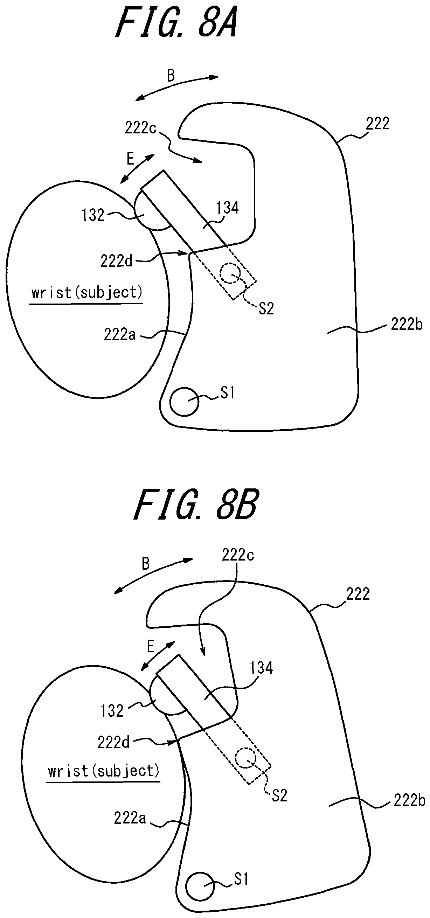

After bringing the pulse pad 132 to be in contact with the skin over the radial artery of his/her wrist, as illustrated in FIG. 8A, the subject pulls the other end 210b of the attaching portion 210 so as to fix the wrist to the electronic device 100 of Example 2. Here, since the exterior portion 222 is configured rotatably in the direction of the arrow B in FIG. 4, when the subject pulls the attaching portion 210, the exterior portion 222 rotates about the shaft S1, and the upper end side is displaced in the y-axis negative direction. That is, as illustrated in FIG. 8B, the upper end side of the exterior portion 222 is displaced in the y-axis negative direction. Since the first arm 134 is connected to the second arm 135 through the elastic body 140, when the upper end side of the exterior portion 222 is displaced in the y-axis negative direction, the pulse pad 132 is biased to the radial artery side by the elastic force of the elastic body 140, which facilitates the pulse pad 132 to catch the change in pulsation more reliably. Thus, according to the electronic device 100 of Example 2, measurement accuracy is improved.

The rotation direction of the exterior portion 222 (the direction indicated by the arrow B) and the rotation direction of the first arm 134 (the direction indicated by the arrow E) may be substantially parallel to each other. The closer the rotation direction of the exterior portion 222 and the rotation direction of the first arm 134 are parallel, when the upper end side of the exterior portion 222 is displaced in the y-axis negative direction, the elastic force of the elastic body 140 is efficiently applied to the first arm 134. It is to be noted that the range where the rotation direction of the exterior portion 222 and the rotation direction of the first arm 134 are substantially parallel includes the range that the elastic force of the elastic body 140 is applied to the first arm 134 when the upper end side of the exterior portion 222 is displaced in the y-axis negative direction.

Here, the front side surface 222b of the exterior portion 222 illustrated in FIGS. 8A and 8B is substantially rectangular which is elongated in the vertical direction. The surface 222b has a notch 222c on the upper end side on the side on the y-axis negative direction side. Even if the upper end side of the exterior portion 222 is displaced in the y-axis negative direction as illustrated in FIG. 8B, due to the notch 222c, the surface 222b does not easily come in contact with the skin over the radial artery. Thus, the pulsation of the radial artery can be easily prevented from being brought in contact with the surface 222b and impeded.

Furthermore, as illustrated in FIG. 8B, when the upper end side of the exterior portion 222 is displaced in the y-axis negative direction, the lower end 222d of the notch 222c comes in contact with a position that is different from the position of the radial artery of the wrist. When the end 222d comes in contact with the wrist, the exterior portion 222 is not displaced in the y-axis negative direction beyond the contact position. Thus, the end 222d can prevent the exterior portion 222 from being displaced beyond the predetermined position. If the exterior portion 222 is displaced in the y-axis negative direction beyond the predetermined position, the first arm 134 is strongly biased to the radial artery side by the elastic force of the elastic body 140. As a result, the pulsation of the radial artery is more likely to be impeded. In the electronic device 100 of Example 2, the exterior portion 222 has the end 222d, which can prevent an excessive pressure from being applied from the first arm 134 to the radial artery, and as a result the pulsation of the radial artery is hardly impeded. In this manner, the end 222d acts as a stopper that limits the displaceable range of the exterior portion 222.

In this embodiment, as illustrated in FIGS. 8A and 8B, a rotary shaft S2 of the first arm 134 may be disposed at a position separated from the side on the y-axis negative direction side of the surface 222b. In the case where the rotary shaft S2 is disposed near the side on the y-axis negative direction side of the surface 222b, when the first arm 134 comes in contact with the wrist of the subject, a change due to pulsation of the radial artery may not be accurately caught. When the rotary shaft S2 is disposed separately from the side on the y-axis negative direction side of the surface 222b, the possibility of the first arm 134 coming in contact the wrist can be reduced. In this manner, the first arm 134 can catch the change in pulsation more accurately.

The subject wears the electronic device 100 of Example 2 on his/her wrist by pulling the other end 210b of the attaching portion 210 and in that state fixing the attaching portion 210 to the fixing mechanism of the fixing portion 212. In a state thus attached to the wrist, the first arm 134 is displaced in the direction indicated by the arrow E according to the change in the pulsation, thus the electronic device 100 of Example 2 measures the pulse wave of the subject.

The above described electronic devices 100 according to Example 1 and Example 2 are merely examples of a configuration of the electronic device 100. Accordingly, the electronic device 100 is not limited to those described as Example 1 and Example 2. The electronic device 100 may only have a configuration capable of measuring the pulse wave of the subject.

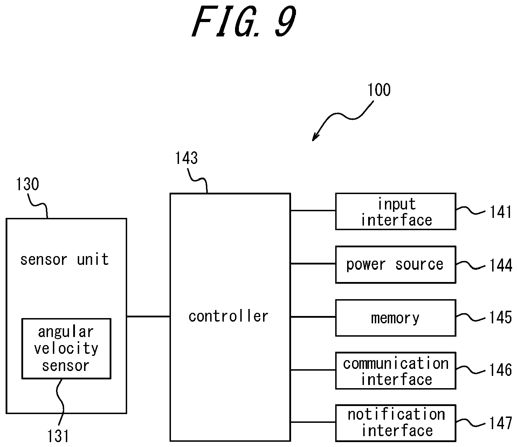

FIG. 9 is a function block diagram of the electronic device 100 according to Example 1 or Example 2. The electronic device 100 includes the sensor unit 130, the input interface 141, the controller 143, the power source 144, the memory 145, the communication interface 146 and the notification interface 147. In the electronic device 100 of Example 1, the controller 143, the power source 144, the memory 145, the communication interface 146 and the notification interface 147 may be included in the measurement unit 120 or the attaching portion 110. In the electronic device 100 of Example 2, the controller 143, the power source 144, the memory 145, the communication interface 146 and the notification interface 147 may be included in the fixing portion 212.

The sensor unit 130 includes the angular velocity sensor 131 and acquires the pulse wave by detecting pulsation from the measured part.

The controller 143 is a processor configured to control and manage the whole electronic device 100 including each function block of the electronic device 100. The controller 143 is also a processor configured to estimate the blood glucose level of the subject from the acquired pulse wave. The controller 143 includes a processor such as a Central Processing Unit (CPU) that executes a program of defining control procedures and a program of estimating the blood glucose level of the subject. These programs are stored in a storage medium such as the memory 145, for example. The controller 143 also estimates the state relating to the glucose or lipid metabolism of the subject based on the index calculated from the pulse wave. The controller 143 may notify data to the notification interface 147.

The power source 144 includes, for example, a lithium-ion battery and a control circuit for charging and discharging the lithium-ion battery, and supplies power to the whole electronic device 100. The power source 144 is not limited to a secondary battery such as a lithium-ion battery or the like, and may be a primary battery such as a button battery or the like.

The memory 145 stores programs and data. The memory 145 may include a semiconductor storage medium and a non-transitory storage medium such as a magnetic storage medium. The memory 145 may include a plurality of types of storage media. The memory 145 may include a combination of a portable storage medium, such as a memory card, an optical disc, or a magneto-optical disc, and an apparatus for reading the storage medium. The memory 145 may include a storage device used as a temporal storage area, such as random access memory (RAM). The memory 145 stores a variety of information and programs for causing the electronic device 100 to operate, or the like, and also acts as a working memory. The memory 145 may, for example, store the measurement result of the pulse wave acquired by the sensor unit 130.

The communication interface 146 transmits and receives a variety of data through wired or wireless communication with an external apparatus. For example, the communication interface 146 communicates with an external apparatus that stores the biological information of the subject to manage the health condition. The communication interface 146 transmits the measurement result of the pulse wave measured by the electronic device 100 and the health condition estimated by the electronic device 100 to the external apparatus.

The notification interface 147 notifies information by sound, vibration, images, or the like. The notification interface 147 may include a speaker, a vibrator, and a display device. The display device may, for example, be a liquid crystal display (LCD), an organic electro-luminescence display (OELD), or an inorganic electro-luminescence display (IELD), and the like. In an embodiment, the notification interface 147 provides notification of the state of the glucose metabolism or lipid metabolism of the subject, for example.

The electronic device 100 according to an embodiment estimates a state of glucose metabolism. In an embodiment, the electronic device 100 estimates the blood glucose level as a state of glucose metabolism.

The electronic device 100 estimates the blood glucose level of the subject using an estimation formula created by regression analysis, for example. The electronic device 100 stores, in advance, estimation formulas for estimating the blood glucose level based on the pulse wave in the memory 145, for example. The electronic device 100 estimates the blood glucose level using these estimation formulas.

Here, the estimation theory relating to estimation of the blood glucose level based on the pulse wave is described. As a result of an increase in the blood glucose level after meal, the blood fluidity is reduced (viscosity is increased), blood vessels dilate, and the amount of circulating blood is increased. Vascular dynamics and hemodynamics are determined so as to balance these states. The reduction in blood fluidity is caused, for example, by an increase in the viscosity of blood plasma or a reduction in the deformability of red blood cells. Further, dilation of blood vessels is caused by secretion of insulin, secretion of digestive hormones, a rise in body temperature, and the like. When blood vessels dilate, pulse rate is increased to suppress a reduction in blood pressure. Further, the increase in the amount of circulating blood compensates for blood consumption for digestion and absorption. Changes in vascular dynamics and hemodynamics before and after meal due to these factors are also reflected in the pulse wave. In this manner, the blood pressure level and the pulse wave change before and after meals. Therefore, the electronic device 100 can estimate the blood glucose level based on the pulse wave.

Estimation formulas for estimating the blood glucose level based on the above described estimation theory can be created by performing regression analysis based on the sample data of the preprandial pulse wave and postprandial blood glucose level acquired from a plurality of subjects. At the time of estimation, the blood glucose level of the subject can be estimated by applying the created estimation formulas to the index based on the pulse wave of the subject. In creation of an estimation formula, in particular, the blood glucose level of the subject, which is an object to be tested, can be estimated by creating an estimation formula by performing regression analysis using sample data in which variation of the blood glucose level is close to the normal distribution. An estimation formula may be created by the Partial Least Squares (PLS) regression analysis, for example. In the PLS regression analysis, the regression coefficient matrix is calculated using the covariance between the objective variable (feature quantity to be estimated) and the explanatory variable (feature quantity to be used for estimation), and by performing multiple regression analysis by adding to the variables in order from the component with the highest correlation between the variables.

Herein, preprandial refers to before the subject has a meal, that is, when the subject is fasting, for example. Herein postprandial refers to after the subject has a meal, that is, the time in the predetermined hours after a meal when the effect of the meal is reflected in the blood. As described in this embodiment, when the electronic device 100 estimates the blood glucose level, the postprandial refers to the time when the blood glucose level rises (for example, approximately one hour after the start of the meal).

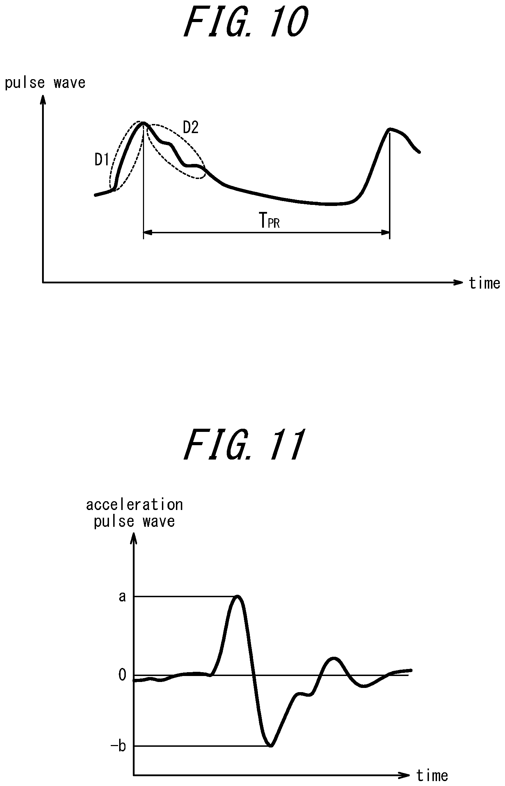

FIG. 10 is a diagram illustrating an example of an estimation method based on a change in pulse wave and illustrates an example of pulse wave. The estimation formulas for estimating the blood glucose level are created using regression analysis relating to an age, an index SI indicating the rising of a pulse wave (rising index), the augmentation index (AI), and the pulse rate (PR).

The rising index SI is derived based on the waveform indicated in the area D1 in FIG. 10. Specifically, the rising index SI is the ratio of the first local minimum to the first local maximum in the acceleration pulse wave derived by the second derivative of the pulse wave. For example, for the acceleration pulse wave illustrated as an example in FIG. 11, the rising index SI is expressed as -b/a. The rising index SI is decreased by dilation (relaxation) of blood vessels caused by a reduction in the blood fluidity, secretion of insulin, and an increase in body temperature, or the like, after meal.

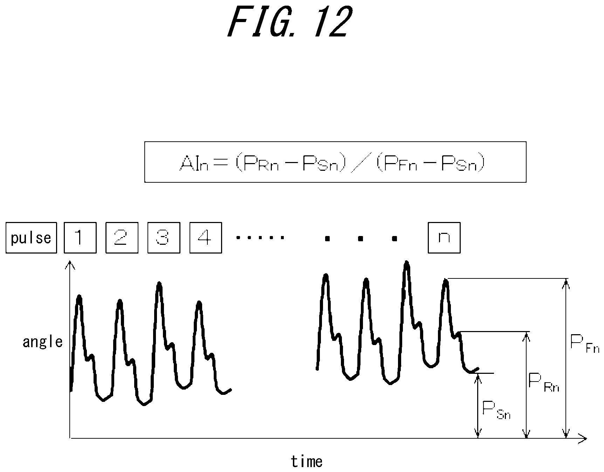

AI is an index represented as the ratio between the magnitude of the forward wave and the reflected wave of the pulse wave. A derivative method of AI will be described with reference to FIG. 12. FIG. 12 is a diagram illustrating an example of pulse waves acquired at the wrist using the electronic device 100. FIG. 12 illustrates the case where the angular velocity sensor 131 is used as the means for detecting the pulsation. FIG. 12 is a time integration of the angular velocity acquired by the angular velocity sensor 131. In FIG. 12, the horizontal axis represents the time and the vertical axis represents the angle. Since the acquired pulse wave may, for example, include noise caused by body movement of the subject, the pulse wave may be corrected by a filter that removes the direct current (DC) component, so as to extract only the pulsation component.

Propagation of the pulse wave is a phenomenon in which pulsation due to blood pumped from the heart is transmitted through artery walls or blood. The pulsation due to blood pumped from the heart reaches the peripheries of limbs as a forward wave, a portion of which is reflected at locations such as where a blood vessel branches, or where the diameter of a blood vessel changes, and returns as a reflected wave. The AI is the result of dividing the magnitude of the reflected wave by the magnitude of the forward wave and is represented as AIn = (PRn - PSn)/(PFn - PSn). Here, the AIn is the AI for each pulse. The AI may, for example, be calculated by measuring the pulse wave for several seconds and calculating the average AIave of the AIn (n = an integer from 1 to n) for each pulse beat. The AI is derived from the waveform indicated in area D2 in FIG. 10. The AI is decreased due to a reduction in the blood fluidity after meal and dilation of the blood vessels due to an increase in the body temperatures, or the like.

The pulse rate PR is derived based on the period TPR of the pulse wave illustrated in FIG. 10. The pulse rate PR rises after meal.

The electronic device 100 can estimate the blood glucose level by an estimation formula created based on the age, the rising index SI, the AI and the pulse rate PR.

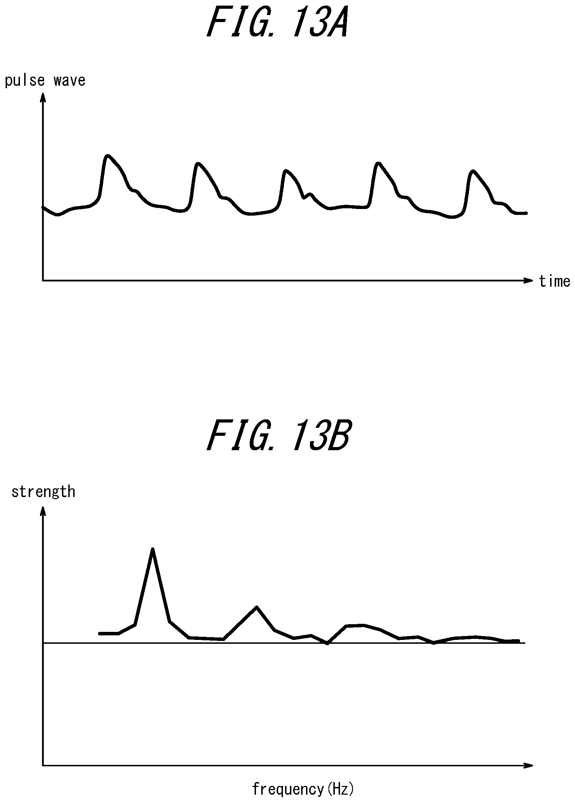

FIGS. 13A and 13B are diagrams illustrating another example of an estimation method based on the change in the pulse wave. FIG. 13A illustrates a pulse wave and FIG. 13B illustrates the result of performing a Fast Fourier Transform (FFT) on the pulse wave in FIG. 13A. An estimation formula for estimating the blood glucose level is, for example, created by regression analysis related to a fundamental wave and harmonic wave component (Fourier coefficients) that are derived by the FFT, for example. The peak value as the result of the FFT illustrated in FIG. 13B changes according to the change in the waveform of the pulse wave. Therefore, the blood glucose level can be estimated using an estimation formula created based on the Fourier coefficients.

The electronic device 100 estimates the blood glucose level of the subject using an estimation formula and based on the above described rising index SI, AI, pulse rate PR, Fourier coefficients and the like.

Here, a method of creating an estimation formula used in the case where the electronic device 100 estimates the blood glucose level of the subject will be described. The estimation formula may not be created by the electronic device 100, and may be created in advance using another computer or the like. Herein the device that creates an estimation formula is referred to as an estimation formula creation apparatus. The created estimation formula is, for example, stored in the memory 145 in advance, before the subject estimates the blood glucose level using the electronic device 100.

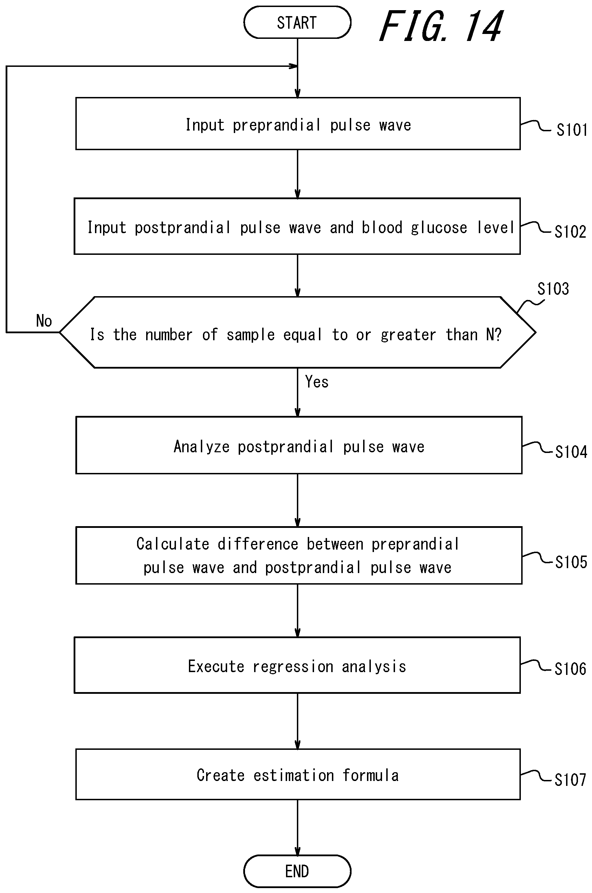

FIG. 14 is a flowchart for creating an estimation formula used by the electronic device 100. The estimation formula is created by performing regression analysis based on the sample data acquired by measuring the preprandial and postprandial pulse wave of the subject by using a pulse wave meter and measuring the postprandial blood glucose level of the subject by using a blood glucose meter. The acquired sample data are not limited to those acquired after meal. It suffices if the data is acquired at times when variation in the blood glucose level is large.

In creation of an estimation formula, first, the information on the preprandial pulse wave of the subject measured by a pulse wave meter is input into the estimation formula creation apparatus (step S101).

Further, the information on the postprandial pulse wave of the subject measured by a pulse wave meter and the information on the postprandial blood glucose level of the subject measured by a blood glucose meter are input to the estimation formula creation apparatus (step S102). The blood glucose level input in step S102 is measured by a blood glucose meter by collecting a blood sample. In step S101 or S102, the age of the subject of each sample data may also be input.

The estimation formula creation apparatus determines whether the number of samples in the sample data input in steps S101 and S102 is equal to or greater than the number of samples, N, that is sufficient for performing the regression analysis (step S103). The number of samples, N, may be determined as appropriate, and may be 100, for example. When determining that the number of samples is smaller than N (in the case of "No"), the estimation formula creation apparatus repeats steps S101 and S102 until the number of samples becomes equal to or greater than N. On the other hand, when determining that the number of samples is greater than or equal to N (in the case of "Yes"), the estimation formula creation apparatus proceeds the step to S104 and calculates the estimation formula.

During calculation of the estimation formula, the estimation formula creation apparatus analyzes the input postprandial pulse wave (step S104). In this embodiment, the estimation formula creation apparatus analyzes the postprandial pulse wave rising index SI, AI and pulse rate PR. The estimation formula creation apparatus may perform FFT analysis as an analysis of the pulse wave.

Further, in calculation of an estimation formula, the estimation formula creation apparatus calculates a difference between the input preprandial pulse wave and the postprandial pulse wave (step S 105). The difference between the preprandial pulse wave and the postprandial pulse wave is a change in the value relating to the pulse wave. The difference is a change in the specific index value relating to the pulse, or a difference between the specific index values relating to the pulse. For example, the difference between the preprandial pulse wave and the postprandial pulse wave is a change in the pulse rate. That is, in step S105, the estimation formula creation apparatus calculates the preprandial and postprandial pulse rates, and calculates the amount of change in the pulse rate by subtracting the preprandial pulse rate from the postprandial pulse rate.

The estimation formula creation apparatus then executes the regression analysis (step S106). The objective variable in the regression analysis is a postprandial blood glucose level. Further, the explanatory variables in the regression analysis are the age input in step S101 or S102, the postprandial pulse wave rising index SI, the AI, and the pulse rate PR analyzed in step S104 and the difference in pulse waves calculated in step S105. It is to be noted that, when the estimation formula creation apparatus performs FFT analysis in step S105, the explanatory variable may be Fourier coefficients calculated as a result of FFT analysis, for example.

The estimation formula creation apparatus creates an estimation formula for estimating the postprandial blood glucose level based on the results of the regression analysis (step S107).

The index for which the pulse wave difference is calculated is not limited to the pulse rate described above. The index for which the pulse wave difference is calculated may be another index relating to the pulse wave, other than the pulse rate. For example, the index for which the pulse wave difference is calculated may include the rising index SI or the AI. Two or more of these indices may be included in the index for which the pulse wave difference is calculated.

It is to be noted that an estimation formula does not necessarily have to be created by the PLS regression analysis. An estimation formula may be created by using other techniques. For example, an estimation formula may be created by the neural network regression analysis.

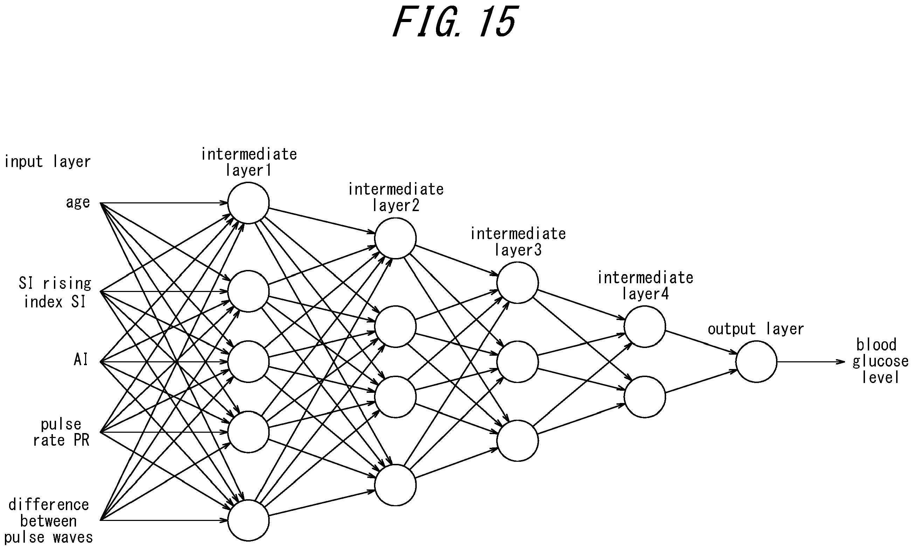

FIG. 15 is a diagram illustrating an example of the neural network regression analysis. FIG. 15 schematically illustrates a neural network in which an input layer is 5 neurons and an output layer is 1 neuron. The 5 neurons of the input layer are the age, the rising index SI, the AI, the pulse rate PR and the pulse wave difference. The 1 neuron of the output layer is the blood glucose level. The neural network illustrated in FIG. 15 has five intermediate layers such as an intermediate layer 1, an intermediate layer 2, an intermediate layer 3, an intermediate layer 4 and an intermediate layer 5 between the input layer and the output layer. The number of nodes of the intermediate layer 1, the intermediate layer 2, the intermediate layer 3, the intermediate layer 4 and the intermediate layer 5 are 5, 4, 3, 2 and 1, respectively. For each node of the intermediate layer, weighting is performed on each component of the data output from the layer one layer before, a sum thereof is taken and is input. In each node of the intermediate layer, a value obtained by performing a predetermined operation (bias) with respect to the input data is output. In the neural network regression analysis, the estimated value of output is compared with the correct value of output by the error back-propagation method, and the weights and the biases in the network are adjusted so that these differences are minimized. In this manner, an estimation formula can also be created by the neural network regression analysis.

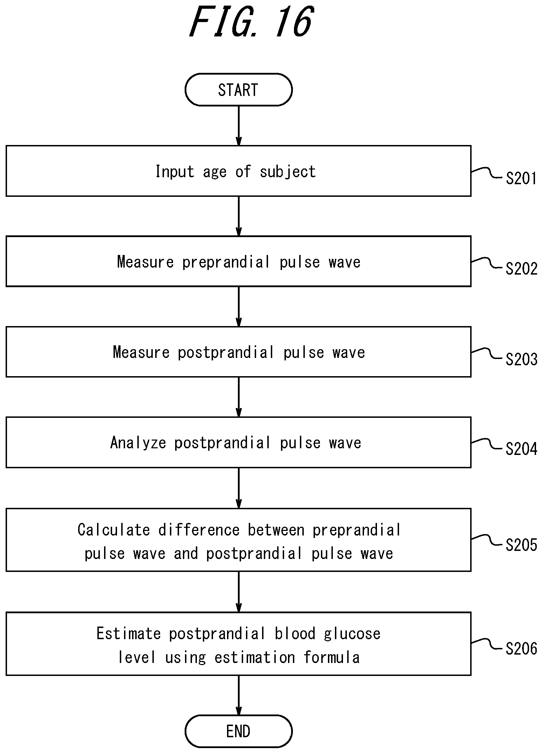

Next, an example of an estimation flow of the blood glucose level of the subject using an estimation formula will be described. FIG. 16 is a flowchart of estimating the postprandial blood glucose level of the subject using a created estimation formula.

First, the electronic device 100 inputs an age of the subject based on the operation of the input interface 141 by the subject (step S201).

The electronic device 100 measures the preprandial pulse wave of the subject based on the operation by the subject (step S202).

The electronic device 100 then measures the postprandial pulse wave of the subject based on the operation by the subject after the subject has meal (step S203).

The electronic device 100 analyzes the measured postprandial pulse wave (step S204). Specifically, the electronic device 100 analyzes the rising index SI, the AI and the pulse rate PR relating to the measured postprandial pulse wave, for example.

Further, the electronic device 100 calculates a difference between the preprandial pulse wave and the postprandial pulse wave (step S205). The difference between the preprandial pulse wave and the postprandial pulse wave may be an index similar to that calculated in creation of an estimation formula. For example, when a change in the pulse rate is calculated in creation of an estimation formula as illustrated in FIG. 14, the difference in pulse waves calculated here by the electronic device 100 may be a change in the pulse rate. That is, in step S205, the electronic device 100 calculates, for example, the preprandial and postprandial pulse rate, and calculates the difference between the calculated preprandial and postprandial pulse rates.

The electronic device 100 estimates the postprandial blood glucose level of the subject by applying the age of the subject whose input is accepted in step S201, the rising index SI, the AI and the pulse rate PR relating to the postprandial pulse rate analyzed in step S204, and the difference between pulse waves calculated in step S205 to the estimation formula (step S206). The estimated postprandial blood glucose level is notified from the notification interface 147 of the electronic device 100 to the subject, for example.

As described above, according to the electronic device 100 of this embodiment, the postprandial blood glucose level of the subject can be estimated based on the pulse wave difference and the postprandial pulse wave of the subject. Thus, according to the electronic device 100, the postprandial blood glucose level can be estimated non-invasively in a short time. In this manner, according to the electronic device 100, the health condition of the subject can be easily estimated.

It is to be noted that the electronic device 100 may estimate not only the postprandial blood glucose level but also the blood glucose level of the subject at any timing. The electronic device 100 can also estimate the blood glucose level at any timing non-invasively in a short time.

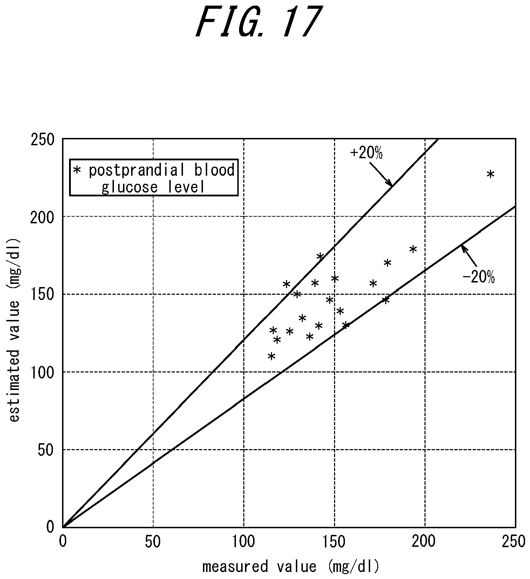

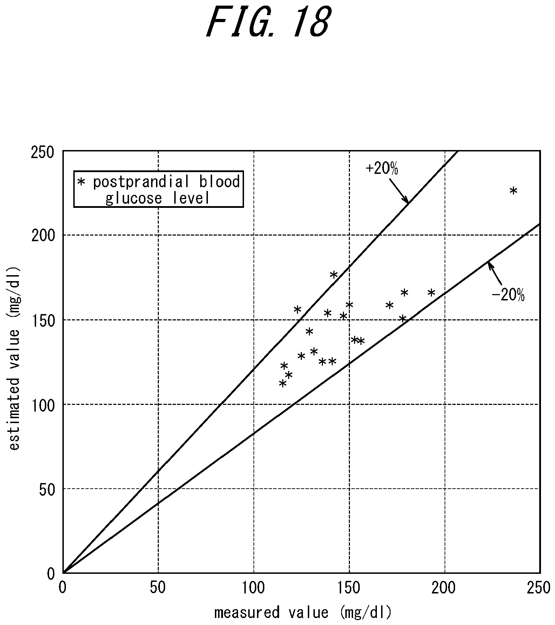

FIGS. 17 and 18 are diagrams each illustrating a comparison between the estimated postprandial blood glucose level and the measured postprandial blood glucose level. FIGS. 17 and 18 are diagrams each illustrating a comparison between the postprandial blood glucose level of the subject estimated based on the preprandial and the postprandial pulse waves of the subject acquired by the sensor unit 130 and the measured postprandial blood glucose level. The estimated value of the postprandial blood glucose level in FIG. 17 is calculated using an estimation formula created by not including a pulse wave difference as an explanatory variable. That is, the estimation formula used for estimating the postprandial blood glucose level in FIG. 17 is created by using the age, the postprandial pulse wave rising index SI, the AI and the pulse wave PR as objective variables. Therefore, the estimated postprandial blood glucose level in FIG. 17 is calculated, using an estimation formula, based on the age and the postprandial pulse wave. The estimated postprandial blood glucose level in FIG. 18 is calculated, using an estimation formula described in this embodiment, based on the difference between the preprandial pulse wave and the postprandial pulse wave of the subject. That is, the estimated postprandial blood glucose level in FIG. 18 is calculated using an estimation formula created including a pulse wave difference as an explanatory variable. In each graph illustrated in FIGS. 17 and 18, the horizontal axis shows the measured value of the postprandial blood glucose level and the vertical axis shows the estimated value of the postprandial blood glucose level. The blood glucose level was measured using the Medisafe FIT ® blood glucose meter from Terumo Corporation.

As illustrated in FIGS. 17 and 18, the measured values and the estimated values are mostly included within the range of ±20%. That is, the estimation accuracy by the estimation formula is within 20%. Here, as a result of calculating the correlation coefficient between the measured values and the estimated values in FIGS. 17 and 18, the correlation coefficient for FIG. 17 was 0.817 and that for FIG. 18 was 0.822. That is, it was found that, as compared with the case illustrated in FIG. 17 where the pulse wave difference is not included as an explanatory variable, the correlation coefficient is higher in the case illustrated in FIG. 18 where the pulse wave difference is included as an explanatory variable. This is because, as illustrated in FIG. 17, when the pulse wave difference is not included as an explanatory variable, the feature of an index value relating to individual pulse wave for each subject (e.g. pulse rate) is not considered, whereas, as illustrated in FIG. 18, when the pulse wave difference is included as an explanatory variable, the feature of an index value relating to individual pulse wave for each subject is reflected in the estimation results of postprandial blood glucose level. For example, assuming that the pulse rate of the subject per minute is higher than the average pulse rate per minute by 30%, and when estimating the postprandial blood glucose level of such subject, if the pulse wave difference is not included as an explanatory variable, the postprandial blood glucose level is estimated without reflecting the feature that the pulse rate of the subject is originally higher than the average. Thus there may be a difference between the estimated postprandial blood glucose level and the correct postprandial blood glucose level of the subject. On the other hand, when the pulse wave difference is included as an explanatory variable, the postprandial blood glucose level can be estimated based on the change in the pulse rate of the subject regardless of the original pulse rate of the subject. That is, in this case, the postprandial blood glucose level can be estimated by reflecting the feature of the pulse rate of the subject. Therefore, according to the electronic device 100 of this embodiment, the postprandial blood glucose level can be more accurately estimated according to each subject.

The estimation method of the postprandial blood glucose level by the electronic device 100 is not limited to the above described method. For example, when estimating the postprandial blood glucose level of the subject, the electronic device 100 may select one estimation formula from a plurality of estimation formulas, and estimate the postprandial blood glucose level of the subject using the selected estimation formula. In this case, estimation formulas are created in advance.

For example, estimation formulas may be created according to the content of the meal. The content of the meal may include, for example, the amount and the quality of the meal. The amount of the meal may include, for example, the weight of the meal. The quality of the meal may include, for example, the menu, the material (food) and the cooking method and the like.

The content of the meal may be classified into some categories. For example, the content of the meal may be classified into categories such as noodles, set meal, bowl and the like. The number of estimation formulas may be the same as that of the classified categories of the content of the meal, for example. That is, when the content of the meal is classified into three categories, for example, an estimation formula may be created corresponding to each category. In this case, the number of estimation formulas created is three. The electronic device 100 estimates the postprandial blood glucose level using an estimation formula, out of a plurality of estimation formulas, corresponding to the content of the meal of the subject.

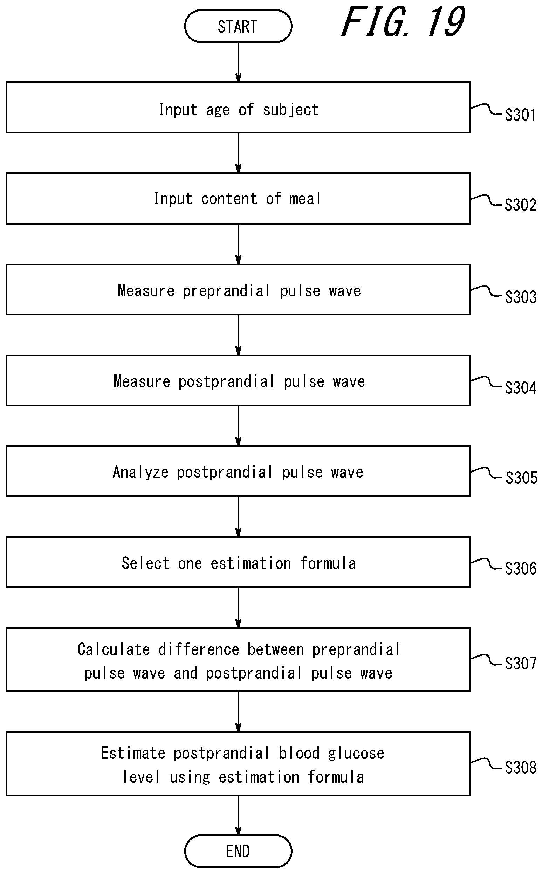

Here, an example of an estimation flow of the blood glucose level of the subject using an estimation formula when a plurality of estimation formulas are created will be described. FIG. 19 is a flowchart for estimating the postprandial blood glucose level of the subject using a plurality of estimation formulas created.

The electronic device 100 inputs the age of the subject based on the operation of the input interface 141 by the subject (step S301).

The electronic device 100 inputs the content of the meal based on the operation of the input interface 141 by the subject (step S302). The electronic device 100 can accept the input of the content of the meal from the subject in a variety of manners. For example, when the electronic device 100 has a display device, the display device displays the content of the meal (e.g. category) that can be selected by the subject. Thus the electronic device 100 allows the subject to select a meal, among the meals displayed, that is closest to the meal the subject is going to eat, and thus may accept an input. For example, the electronic device 100 may accept an input by allowing the subject to describe a content of the meal using the input interface 141. For example, when the electronic device 100 has an imaging unit such as a camera and the like, it may accept an input by allowing the subject to photograph the meal that he/she is going to eat by using the imaging unit. In this case, the electronic device 100 may estimate the content of the meal by analyzing an accepted image, for example.

The electronic device 100 measures the preprandial pulse wave of the subject based on the operation by the subject (step S303).

Then, the electronic device 100 measures, after the subject has meal, the postprandial pulse wave of the subject based on the operation by the subject (step S304).

The electronic device 100 analyzes the measured postprandial pulse wave (step S305). Specifically, the electronic device 100 analyzes the rising index SI, the AI and the pulse rate PR relating to the measured pulse wave, for example.

The electronic device 100 selects one estimation formula from a plurality of estimation formulas based on the content of the meal accepted in step S302 (step S306). The electronic device 100 selects an estimation formula corresponding to a category that is closest to the content of the meal input, for example.

Further, the electronic device 100 calculates the difference between the preprandial pulse wave and the postprandial pulse wave (step S307). For example, the electronic device 100 calculates the preprandial and postprandial pulse rates and calculates the difference between the calculated preprandial pulse wave and the postprandial pulse wave.

The electronic device 100 applies the age of the subject whose input is accepted in step S301, the rising index SI, the AI and the pulse rate PR relating to the postprandial pulse wave analyzed in step S305 and the pulse wave difference calculated in step S307 to the estimation formula to estimate the postprandial blood glucose level of the subject (step S308). The estimated postprandial blood glucose level is notified from the notification interface 147 of the electronic device 100, for example, to the subject.

The change from the preprandial blood glucose level to the postprandial blood glucose level may vary depending on the content of the meal. However, as described above, since the electronic device 100 estimates the postprandial blood glucose level using an estimation formula, out of a plurality of estimation formulas, that corresponds to the content of the meal, the blood glucose level can be estimated more accurately according to the content of the meal.

### (Second Embodiment)

In the first embodiment, the case where the electronic device 100 estimates the postprandial blood glucose level of the subject has been described. In the second embodiment, an example of the case where the electronic device 100 estimates the postprandial lipid level of the subject will be described. Here, the lipid level includes neutral fat, total cholesterol, HDL cholesterol, LDL cholesterol and the like. In the description of this embodiment, the same points as those described in the first embodiment will be omitted as appropriate.

The electronic device 100 previously stores estimation formulas for estimating the lipid level based on the pulse wave in the memory 145, for example. The electronic device 100 estimates the lipid level using these estimation formulas.

The estimation theory relating to the estimation of the lipid level based on the pulse wave is the same as that of the blood glucose level described in the first embodiment. That is, the change in the lipid level in the blood is reflected also in the waveform of the pulse wave. Thus, the electronic device 100 can acquire the pulse wave and estimate the lipid level based on the acquired pulse wave.

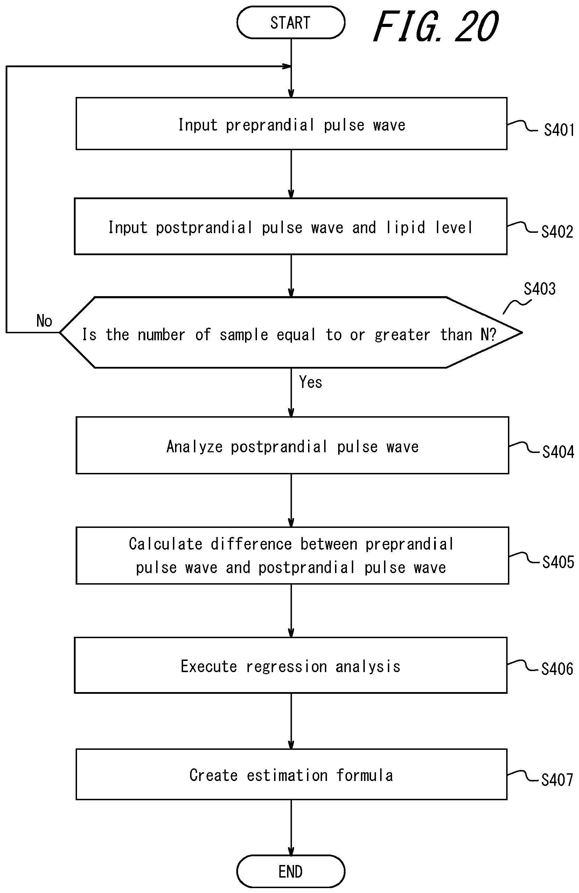

FIG. 20 is a flowchart for creating an estimation formula used by the electronic device 100 according to this embodiment. Also in this embodiment, an estimation formula is created, based on the sample data, by performing a regression analysis such as the PLS regression analysis or the neural network regression analysis, for example. In this embodiment, an estimation formula is created based on the preprandial pulse wave as the sample data. In this embodiment, postprandial may be the time when the lipid level rises after a predetermined time from meal (for example, approximately three hours after the start of the meal). The estimation formula is created in particular by performing regression analysis using sample data in which variation in the lipid levels is close to the normal distribution, and thus the lipid level of the subject to be tested can be estimated at any timing.

In creation of an estimation formula, first, the information on the preprandial pulse wave of the subject measured by a pulse wave meter is input to the estimation formula creation apparatus (step S401).

Further, the information on the postprandial pulse wave of the subject measured by the pulse wave meter and the information on the postprandial lipid level of the subject measured by the lipid measurement apparatus are input to the estimation formula creation apparatus (step S402). The age of the subject of each sample data may also be input in step S401 or S402.

The estimation formula creation apparatus determines whether the number of samples in the sample data input in steps S401 and S402 is equal to or greater than the number of samples, N, that is sufficient for performing the regression analysis (step S403). The number of samples, N, can be determined as appropriate, and may be 100, for example. When the estimation formula creation apparatus determines that the number of samples is less than N (in the case of "No"), it repeats steps S401 and S402 until the number of samples becomes equal to or greater than N. On the other hand, when the estimation formula creation apparatus determines that the number of samples is greater than or equal to N (in the case of "Yes"), it proceeds the step to step S404 and calculates the estimation formula.

In calculation of the estimation formula, the estimation formula creation apparatus analyzes the input preprandial pulse wave (step S404). In this embodiment, the estimation formula creation apparatus analyzes the rising index SI, the AI and the pulse rate PR of the postprandial pulse wave. It is to be noted that the estimation formula creation apparatus may perform FFT analysis as a pulse wave analysis.

Further, in calculation of an estimation formula, the estimation formula creation apparatus calculates the difference between the input preprandial pulse wave and the postprandial pulse wave (step S405). For example, in step S405, the estimation formula creation apparatus calculates the amount of change in the pulse rate by calculating the preprandial and postprandial pulse rate and subtracting the preprandial pulse rate from the postprandial pulse rate.

Then, the estimation formula creation apparatus performs the regression analysis (step S406). An objective variable in the regression analysis is the postprandial lipid level. Further, an explanatory variables in the regression analysis are, for example, the age input in step S401 or S402, the rising index SI, the AI and the pulse rate PR of the postprandial pulse wave analyzed in step S404 and the pulse wave difference calculated in step S405. It is to be noted that, when the estimation formula creation apparatus performs FFT analysis in step S105, the explanatory variable may be Fourier coefficients calculated as a result of FFT analysis, for example.

The estimation formula creation apparatus creates an estimation formula for estimating the postprandial lipid level based on the result of the regression analysis (step S407).

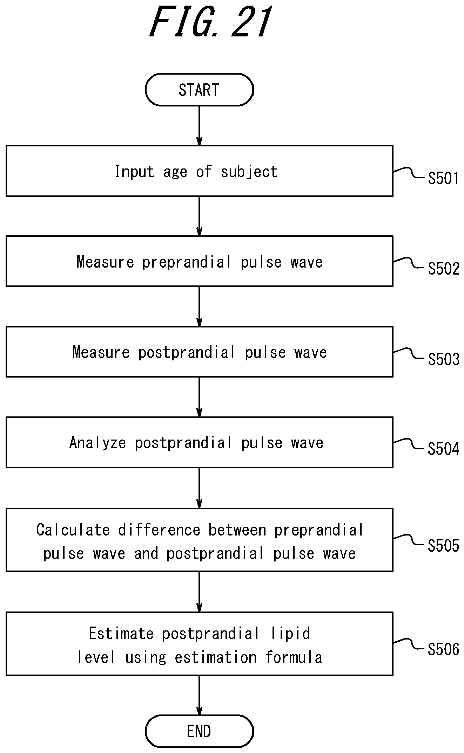

Next, a flow for estimating the lipid level of the subject using an estimation formula will be described. FIG. 21 is a flowchart for estimating the postprandial lipid level of the subject using an estimation formula created by the flow in FIG. 20, for example.

First, the electronic device 100 inputs an age of the subject based on the operation of the input interface 141 by the subject (step S501).

The electronic device 100 inputs the preprandial pulse wave of the subject based on the operation by the subject (step S502).

The electronic device 100 then measures, after the subject has meal, the postprandial pulse wave of the subject based on the operation by the subject (step S503).

The electronic device 100 analyzes the measured postprandial pulse wave (step S504). Specifically, the electronic device 100 analyzes the rising index SI, the AI and the pulse rate PR relating to the measured postprandial pulse wave, for example.

Further, the electronic device 100 calculates the difference between the preprandial pulse wave and the postprandial pulse wave (step S505). For example, in step S505, the electronic device 100 calculates the preprandial and postprandial pulse rates and the difference between the calculated preprandial and postprandial pulse rates.

The electronic device 100 estimates the postprandial lipid level of the subject by applying the age of the subject whose input is accepted in step S501, the rising index SI, the AI and the pulse rate PR relating to the postprandial pulse wave analyzed in step S504, and the pulse wave difference calculated in step S505 to an estimation formula (step S506). The estimated postprandial lipid level is notified from the notification interface 147 of the electronic device 100 to the subject, for example.

As described above, according to the electronic device 100 of this embodiment, the postprandial lipid level of the subject can be estimated based on the pulse wave difference and the postprandial pulse wave of the subject. Thus, according to the electronic device 100, the postprandial lipid level can be estimated non-invasively in a short time. In this manner, according to the electronic device 100, the heath condition of the subject can be estimated easily and conveniently. Further, according to the electronic device 100, the postprandial lipid level can be estimated by reflecting the feature of the pulse rate of the subject. Thus, according to the electronic device 100 of this embodiment, the postprandial lipid level corresponding to each subject can be estimated more accurately.

Also in the case where the lipid level is estimated, as in the example of the case where the blood glucose level is estimated, one estimation formula is selected from a plurality of estimation formulas, and the lipid level may be estimated using the selected estimation formula.

In the above described embodiment, an example of the case where estimations of the blood glucose level and the lipid level are performed by the electronic device 100 has been described. However, estimations of the blood glucose level and the lipid level may not necessarily be performed by the electronic device 100. An example of the case where estimations of the blood glucose level and the lipid level are performed by an apparatus other than the electronic device 100 will be described.

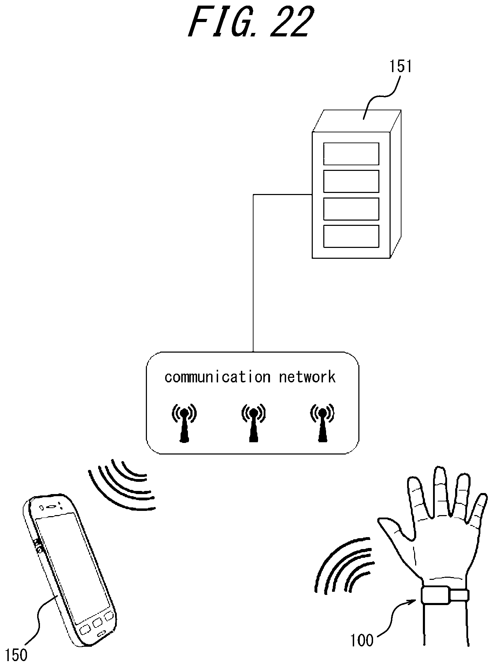

FIG. 22 is a schematic diagram illustrating a schematic configuration of a system according to an embodiment. The system according to the embodiment illustrated in FIG. 22 includes the electronic device 100, an information processor (e.g. a server) 151, a mobile terminal 150 and a communication network. As illustrated in FIG. 22, the pulse wave measured by the electronic device 100 is transmitted to the information processor 151 over the communication network and is stored in the information processor 151 as the personal information of the subject. The information processor 151 compares the pulse wave with the information of the subject acquired in the past or a variety of data base to estimate the blood glucose level or the lipid level of the subject. The information processor 151 may further create appropriate advice for the subject. The information processor 151 replies to the mobile terminal 150 in the subject's possession with estimation results and advice. The mobile terminal 150 can establish a system to provide notification of received estimation results and advice through the display of the mobile terminal 150. By using the communication function of the electronic device 100, information from a plurality of users can be collected on the information processor 151. Thus the estimation accuracy is further increased. Further, since the mobile terminal 150 is used as notification means, the electronic device 100 does not require the notification interface 147 any longer, and can be further reduced in size. Further, since the blood glucose level or the lipid level of the subject is estimated by the information processor 151, the calculation load on the controller 143 of the electronic device 100 can be reduced. Further, since the subject's information acquired in the past can be stored on the information processor 151, load on the memory 145 of the electronic device 100 can be reduced. Therefore, the electronic device 100 can be further reduced in size and in complexity. Further, the processing speed for calculation is also improved.

In the system according to this embodiment, the electronic device 100 and the mobile terminal 150 are connected over the communication network through the information processor 151. However, the system according to this disclosure is not limited to the above described configuration. The electronic device 100 and the mobile terminal 150 may be connected directly over the communication network without use of the information processor 151.

In order to completely and clearly disclose this disclosure, characteristic examples have been described. However, the appended claims are not limited to the above embodiments and are to be constructed as embodying all of the possible modifications and alternate configurations that a person of ordinary skill in the art could have created within the scope of the fundamental features indicated in this specification.

For example, in the above described embodiments, the case where the sensor unit 130 is provided with the angular velocity sensor 131 has been described. However, the electronic device 100 according to this disclosure is not limited thereto. The sensor unit 130 may be provided with an optical pulse wave sensor constituted by a light emitting portion and a light receiving portion or may be provided with a pressure sensor. Further, the electronic device 100 is not limited to be worn on the wrist. It suffices for the sensor unit 130 to be placed on an artery, such as on the neck, ankle, thigh, ear, or the like.

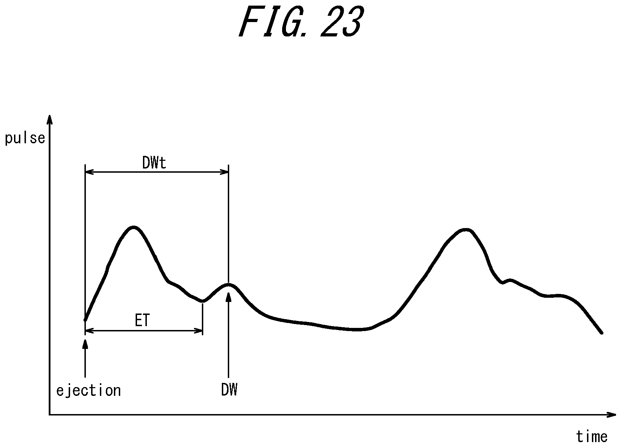

Further, for example, in the above described embodiments, although the explanatory variables of the regression analysis have been described as being the age, the rising index SI, the AI and the pulse rate PR, the explanatory variables may not include all of these four or may include variables other than these four. For example, the explanatory variables may include a gender or an index determined based on a velocity pulse wave derived by differentiating a gender or a pulse wave once. For example, the explanatory variables may include an index determined based on the pulse. The index determined based on the pulse may include, for example, the ejection time (ET) or the time DWt from the ventricular ejection to the dicrotic wave (DW) illustrated as an example in FIG. 23. Further, for example, the explanatory variables may include a fasting blood glucose level (e.g. a blood glucose level measured by blood sampling, a blood glucose level measured in advance at the time of a medical checkup, or the like).

In the above described embodiments, it has been described that an estimation formula is created using the preprandial and postprandial pulse waves of the subject and the postprandial blood glucose level or lipid level of the subject. Here, the subject may be a subject that causes the electronic device 100 to estimate the blood glucose level or the lipid level. That is, in this case, the estimation formula is created by using the preprandial and postprandial pulse wave of the subject and the postprandial blood glucose level or lipid level of the subject.

In the above described embodiments, the controller 143 has been described to estimate the postprandial blood glucose level or lipid level of the subject based on the difference between the preprandial pulse wave and the postprandial pulse wave of the subject acquired by the sensor unit 130. However, the controller 143 may not necessarily estimate the postprandial blood glucose level or lipid level of the subject based on the difference between the preprandial pulse wave and the postprandial pulse waves of the subject. For example, the controller 143 may estimate, based on the difference between the preprandial pulse wave and the pulse wave at a predetermined timing, the blood glucose level or the lipid level of the timing at the predetermined timing. The predetermined timing may be determined as appropriate. The predetermined timing may be a timing at which the change in the blood glucose level or the lipid level is larger than those of before meal.

### REFERENCE SIGNS LIST

- 100: Electronic device
- 110,: 210Attaching portion
- 111, 225: Opening
- 120, 220: Measurement unit
- 120a: Back surface
- 120b: Surface
- 130: Sensor unit
- 131: Angular velocity sensor
- 132: Pulse pad
- 133, 224: Shaft
- 134: First arm
- 135: Second arm
- 140: Elastic body
- 141: Input interface
- 143: Controller
- 144: Power source
- 145: Memory
- 146: Communication interface
- 147: Notification interface
- 150: Mobile terminal
- 151: Information processor
- 211: Base portion
- 212: Fixing portion
- 221: Body portion
- 222: Exterior portion
- 222a: Contact surface
- 222b: Surface
- 222c: Notch
- 222d: End
- 223: Coupling portion

## Claims

1. An electronic device, comprising:
a sensor unit configured to acquire a pulse wave of a subject; and
a controller configured to estimate, using an estimation formula created based on a preprandial pulse wave and a postprandial pulse wave and blood glucose level, a postprandial blood glucose level of the subject based on a preprandial pulse wave and a postprandial pulse wave of the subject acquired by the sensor unit.

2. The electronic device according to claim 1, wherein a difference between the preprandial pulse wave and the postprandial pulse wave is a change in a value of an index relating to a pulse wave.

3. The electronic device according to claim 2, wherein the index relating to a pulse wave includes at least any one of pulse rate, rising index SI and AI.

4. The electronic device according to claim 1, wherein the controller estimates the postprandial blood glucose level using an estimation formula, out of a plurality of estimation formulas, corresponding to a content of meal of the subject.

5. The electronic device according to claim 4, wherein each of the estimation formulas corresponds to classification of the content of meal.

6. The electronic device according to claim 1, wherein the estimation formula is created by a PLS regression analysis or a neural network regression analysis.

7. An electronic device, comprising:
a sensor unit configured to acquire a pulse wave of a subject; and
a controller configured to estimate, using an estimation formula created based on a preprandial pulse wave and a postprandial pulse wave and lipid level, a postprandial lipid level of the subject based on a difference between preprandial pulse wave and a postprandial pulse wave of the subject acquired by the sensor unit.

8. An estimation system comprising an electronic device and an information processor communicatively connected to each other, wherein
the electronic device includes a sensor unit configured to acquire a pulse wave of a subject; and
the information processor includes a controller configured to estimate, using an estimation formula created based on a preprandial pulse wave and a postprandial pulse wave and blood glucose level, a postprandial blood glucose level of the subject based on a preprandial pulse wave and a postprandial pulse wave of the subject acquired by the sensor unit.

9. An estimation system comprising an electronic device and an information processor communicatively connected to each other, wherein
the electronic device includes a sensor unit configured to acquire a pulse wave of a subject; and
the information processor includes a controller configured to estimate, using an estimation formula created based on a preprandial pulse wave and a postprandial pulse wave and lipid level, a postprandial lipid level of the subject based on a preprandial pulse wave and a postprandial pulse wave of the subject acquired by the sensor unit.

10. An estimation method executed by an electronic device, comprising the steps of:
acquiring a pulse wave of a subject; and
estimating, using an estimation formula created based on a preprandial pulse wave and a postprandial pulse wave and blood glucose level, a postprandial blood glucose level of the subject based on a difference between the preprandial pulse wave and the postprandial pulse wave of the subject acquired.

11. An estimation method executed by an electronic device, comprising the steps of:
acquiring a pulse wave of a subject; and
estimating, using an estimation formula created based on a preprandial pulse wave and a postprandial pulse wave and lipid level, a postprandial lipid level of the subject based on a difference between the preprandial pulse wave and the postprandial pulse wave of the subject acquired.

12. An estimation program configured to cause an electronic device to execute the steps of:
acquiring a pulse wave of a subject; and
estimating, using an estimation formula created based on a preprandial pulse wave and a postprandial pulse wave and blood glucose level, a postprandial blood glucose level of the subject based on a difference between the preprandial pulse wave and the postprandial pulse wave of the subject acquired.

13. An estimation program configured to cause an electronic device to execute the steps of:
acquiring a pulse wave of a subject; and
estimating, using an estimation formula created based on a preprandial pulse wave and a postprandial pulse wave and lipid level, a postprandial lipid level of the subject based on a difference between the preprandial pulse wave and the postprandial pulse wave of the subject acquired.

14. An electronic device, comprising:
a sensor unit configured to acquire a pulse wave of a subject; and
a controller configured to estimate, using an estimation formula created based on a preprandial pulse wave and a postprandial pulse wave and blood glucose level of the subject, a blood glucose level of the subject based on a difference between a preprandial pulse wave and a pulse wave at any timing of the subject acquired by the sensor unit.

15. An electronic device, comprising:
a sensor unit configured to acquire a pulse wave of a subject; and
a controller configured to estimate, using an estimation formula created based on a preprandial pulse wave and a postprandial pulse wave and lipid level of the subject, a lipid level of the subject based on a difference between a preprandial pulse wave and a pulse wave at any timing of the subject acquired by the sensor unit.
